(19) **Europäisches Patentamt / European Patent Office / Office européen des brevets**

(11) **EP 4 722 377 A1**

(12) **EUROPEAN PATENT APPLICATION**
published in accordance with Art. 153(4) EPC

(43) Date of publication:
08.04.2026 Bulletin 2026/15

(21) Application number: 24815600.2

(22) Date of filing: 31.05.2024

(51) International Patent Classification (IPC):
*C12P 21/02* $^{(2006.01)}$     *C12N 15/53* $^{(2006.01)}$
*C12P 21/08* $^{(2006.01)}$

(52) Cooperative Patent Classification (CPC):
C07K 16/00; C12N 9/0004; C12P 21/02

(86) International application number:
PCT/JP2024/019990

(87) International publication number:
WO 2024/248120 (05.12.2024 Gazette 2024/49)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 31.05.2023 JP 2023090290

(71) Applicant: FUJIFILM Corporation
Tokyo 106-8620 (JP)

(72) Inventors:
• NAKAI, Shinichi
Kanagawa 258-8577 (JP)
• INADA, Atsushi
Kanagawa 258-8577 (JP)
• MATSUURA, Tatsuya
Kanagawa 258-8577 (JP)

(74) Representative: Hoffmann Eitle
Patent- und Rechtsanwälte PartmbB
Arabellastraße 30
81925 München (DE)

Remarks:
The complete document including Reference
Table(s) and the Sequence Listing(s) can be
downloaded from the EPO website

(54) **METHOD FOR PRODUCING PRODUCT**

(57) An object of the present invention is to provide a method in which a proliferation capacity of cells is maintained and a product having high quality is obtained with high productivity over a long period of time, in a production method for a product by cell culture.

According to the present invention, there is provided A production method for a product, including: performing perfusion culture of cells in a culture solution, in which a perfusion ratio P (vvd) in the perfusion culture, a cell density V ($\times 10^6$ cells/ml) in the culture solution, and X, which is a ratio of an expression level of an isovaleryl-CoA dehydrogenase gene Ivd to an expression level of a glycerol-3-phosphate dehydrogenase gene Gpdh in the culture solution, satisfy $1.0 \times 10^{-5} \leq X \times (P/V) \leq 1.0 \times 10^{-4}$.

EP 4 722 377 A1

**Description**

**Technical Field**

The present invention relates to a production method for a product, including performing perfusion culture of cells in a culture solution.

**Background Art**

Cell culture is carried out for the intended purpose, for example, increasing the number of cells having useful properties or causing cells to produce a product. In producing a biopharmaceutical product such as antibodies, a perfusion culture method, in which a culture solution is continuously filtered and discharged while a fresh medium containing nutrients is continuously fed to a culture tank, is often used to improve productivity of antibodies.

For example, Patent Document 1 discloses a production method for a double-specific antibody product, the method including (i) a step of supplying at least one kind of mammalian cell capable of expressing a double-specific antibody product in a perfusion bioreactor, (ii) a step of proliferating a mammalian cell culture at a first perfusion amount until a set value of a viable cell density is reached, and (iii) a step of maintaining perfusion culture at a second perfusion amount where the step is such that the concentration of the double-specific antibody product in the bioreactor is maintained to be lower than a threshold value.

Patent Document 2 discloses culturing an animal cell having a gene encoding a target protein and a foreign gene encoding an isovaleryl-CoA dehydrogenase, in which the isovaleryl-CoA dehydrogenase is overexpressed, to produce the target protein.

**Prior Art Documents**

**Patent Documents**

Patent Document 1: JP2021-505182A
Patent Document 2: WO2019/117136A

**Summary of Invention**

**Object to be solved by the invention**

Perfusion culture has attracted attention as a method for improving productivity of antibody drugs. In the perfusion culture, it is important to perform culture while maintaining a cell density substantially constant in order to obtain an antibody having stable quality over a long period of time. However, there is a problem in that a proliferation capacity of the cells is reduced during the culture, the cell density cannot be maintained, the cells die in the middle, and the quality of the antibody is reduced or productivity is reduced. An object to be achieved by the present invention is to provide a method in which a proliferation capacity of cells is maintained and a product having high quality is obtained with high productivity over a long period of time, in a production method for a product by cell culture.

**Means for solving the object**

As a result of diligent studies to achieve the above objects, the inventors of the present invention found that, in the perfusion culture, by controlling an expression level of an isovaleryl-CoA dehydrogenase gene (Ivd) per cell, a cell density in culture, and a perfusion ratio to satisfy a predetermined relational expression, the product having high quality can be obtained with high productivity over a long period of time while maintaining the proliferation capacity, whereby the present invention was completed.

That is, according to the present invention, the following inventions are provided.

<1> A production method for a product, comprising:

performing perfusion culture of cells in a culture solution,
in which a perfusion ratio P (vvd) in the perfusion culture,
a cell density V ($\times 10^6$ cells/ml) in the culture solution, and

X, which is a ratio of an expression level of an isovaleryl-CoA dehydrogenase gene Ivd to an expression level of a glycerol-3-phosphate dehydrogenase gene Gpdh in the culture solution, satisfy

$$1.0 \times 10^{-5} \le X \times (P/V) \le 1.0 \times 10^{-4}.$$

<2> The production method for a product according to <1>, in which an isovaleric acid concentration in the culture solution is 10 μmol/L or more and 1,000 μmol/L or less.

<3> The production method for a product according to <1> or <2>, in which X is 0.0010 or more and 0.0100 or less.

<4> The production method for a product according to any one of <1> to <3>, in which the perfusion culture includes producing the product in a certain period while maintaining a target viable cell density substantially constant after reaching the viable cell density in the perfusion culture.

<5> The production method for a product according to <4>, in which the viable cell density maintained substantially constant is $30 \times 10^6$ cells/ml or more and $400 \times 10^6$ cells/ml or less.

<6> The production method for a product according to <4> or <5>, in which the perfusion ratio is 0.3 or more and 5.0 or less in a period in which the viable cell density is maintained substantially constant.

<7> The production method for a product according to any one of <4> to <6>, in which cell bleeding of discharging a culture solution containing cells to an outside of a system and adding a culture medium is performed to maintain the viable cell density substantially constant.

<8> The production method for a product according to <7>, in which a cell bleeding ratio in the cell bleeding is 0.10 to 0.80 vvd.

<9> The production method for a product according to any one of <1> to <8>, in which a period of the perfusion culture is 14 days or more and 200 days or less.

<10> The production method for a product according to any one of <1> to <9>, in which the isovaleryl-CoA dehydrogenase gene Ivd is an endogenous gene.

<11> The production method for a product according to any one of <1> to <10>, in which the cell is a CHO cell.

<12> The production method for a product according to any one of <1> to <11>, in which the product is a protein.

<13> The production method for a product according to <12>, in which the protein is an antibody.

<14> A production method for a product, comprising:

a screening step of selecting cells in which a ratio of an expression level of an isovaleryl-CoA dehydrogenase gene Ivd to an expression level of a glycerol-3-phosphate dehydrogenase gene Gpdh is 0.0010 or more and 0.0100 or less; and
a step of culturing the cells selected in the screening step,
in which expression of the isovaleryl-CoA dehydrogenase gene Ivd of the cells is endogenous gene expression.

**Effect of the invention**

According to the production method for a product according to the present invention, it is possible to maintain the proliferation capacity of the cell and obtain the product having high quality with high productivity over a long period of time.

**BRIEF DESCRIPTION OF THE DRAWINGS**

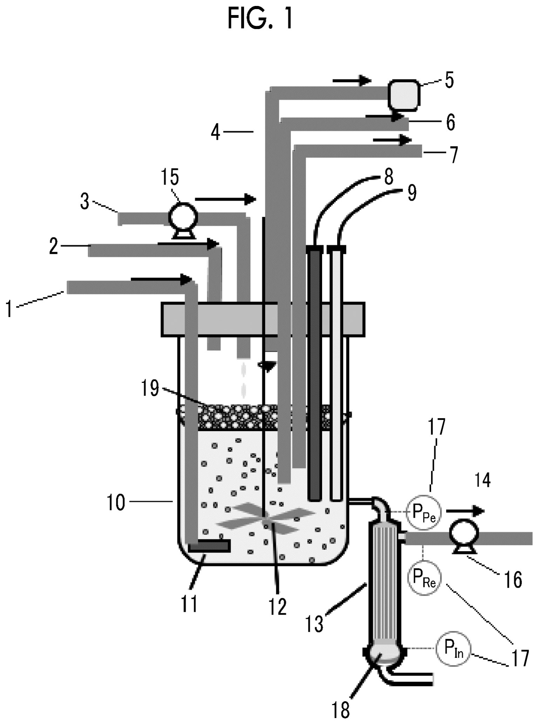

[FIG. 1] FIG. 1 shows a cell culture device.

**Embodiments for carrying out the invention**

Hereinafter, the contents of the present invention will be described in detail. In the present specification, a numerical value range indicated using "to" means a range including numerical values described before and after "to" as a minimum value and a maximum value, respectively.

A production method for a product according to an embodiment of the present invention is a production method for a product, including:

production culturing cells in a culture solution,
in which a perfusion ratio P (vvd) in the perfusion culture,
a cell density V ($\times 10^6$ cells/ml) in the culture solution, and
X, which is a ratio of an expression level of an isovaleryl-CoA dehydrogenase gene Ivd to an expression level of a glycerol-3-phosphate dehydrogenase gene Gpdh in the culture solution, satisfy

$$1.0 \times 10^{-5} \leq X \times (P/V) \leq 1.0 \times 10^{-4}.$$

The production method for a product according to the embodiment of the present invention can be suitably used as a production method for a product by cell culture, which is used for a biopharmaceutical product, regenerative medicine, or the like.

The culture solution contains both cells and a culture supernatant.

The cell density V ($\times 10^6$ cells/ml) in the culture solution can be measured by a live/dead cell analyzer Vi-CELL XR, manufactured by Beckman Coulter, Inc.

X, which is the ratio of the expression level of the isovaleryl-CoA dehydrogenase gene Ivd to the expression level of the glycerol-3-phosphate dehydrogenase gene Gpdh in the culture solution, can be determined by measuring the expression level of Gpdh and the expression level of Ivd, respectively, by real-time polymerase chain reaction (PCR). Specifically, reverse transcription is performed using Total RNA harvested from the cell in the culture solution. Using a primer having homology to the endogenous Ivd and a primer for the glycerol-3-phosphate dehydrogenase Gpdh gene, real-time polymerase chain reaction (PCR) is performed, and the expression level of the Ivd gene can be confirmed using the Gpdh gene for standardization.

The perfusion ratio P (vvd), the cell density V ($\times 10^6$ cells/ml), and X, which is the ratio of the expression level, may be

$$1.0 \times 10^{-5} \leq X \times (P/V) \leq 1.0 \times 10^{-4},$$

but are preferably

$$2.0 \times 10^{-5} \leq X \times (P/V) \leq 8.0 \times 10^{-5},$$

and
more preferably

$$3.0 \times 10^{-5} \leq X \times (P/V) \leq 5.0 \times 10^{-5}.$$

By setting $X \times (P/V)$ to $1.0 \times 10^{-5}$ or more, the increase in the isovaleric acid concentration can be suppressed, and the cell proliferation during the culture can be appropriately maintained. By setting $X \times (P/V)$ to $1.0 \times 10^{-4}$ or less, the culture medium use amount for removing isovaleric acid can be suppressed, and the cost can be reduced.

The isovaleric acid concentration in the culture solution is preferably 10 μmol/L or more and 1,000 μmol/L or less, more preferably 50 μmol/L or more and 800 μmol/L or less, and still more preferably 100 μmol/L or more and 500 μmol/L or less.

The isovaleric acid concentration in the culture solution can be measured by extracting the culture solution from the culture tank, filtering the cells, and then measuring the filtrate using chromatographic separation.

By setting the isovaleric acid concentration in the culture solution to 10 μmol/L or more, the culture medium use amount for removing isovaleric acid can be suppressed, and the cost can be reduced. By setting the isovaleric acid concentration in the culture solution to 1,000 μmol/L or less, the cell proliferation during the culture can be appropriately maintained.

X is preferably 0.0010 or more and 0.0100 or less, more preferably 0.0014 or more and 0.0080 or less, and still more preferably 0.0020 or more and 0.0050 or less. The lower limit value of X is preferably 0.0014, more preferably 0.0020, and still more preferably 0.0025.

In a case where X is 0.0010 or more, the increase in the isovaleric acid concentration can be suppressed, and the cell proliferation during the culture can be appropriately maintained. In a case where X is 0.0100 or less, the Ivd expression can be achieved with the endogenous gene expression, and thus it is not necessary to introduce a foreign gene for the Ivd expression, and the cost can be reduced.

In the present invention, the mode of the method of culturing cells is perfusion culture.

The perfusion culture is a culture method in which a fresh culture medium is added and at the same time the used culture medium is removed. In general, the perfusion culture makes it possible to achieve a high viable cell density. A typical perfusion culture begins with a batch culture start-up lasting 1 or 2 days, thereafter a fresh supply culture medium is added to the culture continuously, stepwise, and/or intermittently, and the used culture medium is removed at the same time. In the perfusion culture, methods such as sedimentation, centrifugation, and filtration can be used to remove the used culture medium while maintaining the viable cell density. The advantage of the perfusion culture is that the culture in which a target protein is produced is maintained for a long period as compared with the batch culture method or the fed-batch culture.

Perfusion may be any form of being continuous, stepwise, intermittent, or a combination thereof. A continuous form is preferable. Animal cells are retained in the culture and the used culture medium that is removed may substantially not include cells or may have much fewer cells than the culture. A product that is expressed by cell culture can be retained in the culture or recovered by the selection of the membrane pore diameter.

The period of the perfusion culture is preferably 5 days or more and 300 days or less, more preferably 14 days or more and 200 days or less, still more preferably 14 days or more and 100 days or less, and still more preferably 20 days or more and 90 days or less. It is preferable for the number of days of the culture to be 5 days or more since the obtained cell number is large and the production amount of a product is large in a case where the cells produce the product. It is preferable for the number of days of the culture to be 300 days or less from the viewpoint of preventing clogging of the filtration membrane used in the perfusion culture and preventing contamination.

The perfusion ratio is not particularly limited; however, it is preferably 0.3 vvd or more and 5.0 vvd or less, more preferably 0.5 vvd or more and 3.0 vvd or less, and still more preferably 0.8 vvd or more and 2.0 vvd or less.

By setting the perfusion ratio to 0.3 or more, the increase in the isovaleric acid concentration can be suppressed, and the cell proliferation during the culture can be appropriately maintained. By setting the perfusion ratio to 5.0 or less, the culture medium use amount can be suppressed, and the cost can be reduced.

The vvd regarding the perfusion ratio means "volume of drained culture solution/volume of culture solution in culture container/day". It is noted that the perfusion ratio described herein is a parameter different from "(iv) driving a pump for draining the culture solution at a rate of 0.3 vvd to 3 vvd in a case where the electrostatic capacity of the culture solution exceeds the target" in one example of the bleeding.

The extraction of the culture solution from the culture container can usually be carried out using a pump, but other available liquid feeding units may be used. The culture solution extracted from the culture container is subjected to treatments such as product recovery and removal of the dead cells. The culture solution extracted from the culture container may be partially discarded or returned to the culture container after treatments such as product recovery and removal of the dead cells. In a case where a loss of the culture medium occurs due to the above treatments, the loss can be compensated, for example, by supplying a fresh culture medium to the culture container.

The perfusion culture preferably includes producing the product in a certain period while maintaining a target viable cell density substantially constant after reaching the viable cell density in the culturing.

The "maintaining the viable cell density substantially constant" means that the viable cell density is maintained within ±20% of the target viable cell density, preferably within ±10%, more preferably within ±5%, and still more preferably within ±3%.

The viable cell density maintained substantially constant is preferably $30 \times 10^6$ cells/ml or more and $400 \times 10^6$ cells/ml or less, more preferably $30 \times 10^6$ cells/ml or more and $300 \times 10^6$ cells/ml or less, still more preferably $80 \times 10^6$ cells/ml or more and $240 \times 10^6$ cells/ml or less, still more preferably $80 \times 10^6$ cells/ml or more and $200 \times 10^6$ cells/ml or less, even still more preferably $80 \times 10^6$ cells/ml or more and $160 \times 10^6$ cells/ml or less, and particularly preferably $100 \times 10^6$ cells/ml or more and $120 \times 10^6$ cells/ml or less. M may be used to denote $10^6$.

By setting the viable cell density to $30 \times 10^6$ cells/ml or more, the productivity of the antibody is increased. By setting the viable cell density to $400 \times 10^6$ cells/ml or less, the increase in the isovaleric acid concentration can be suppressed, and the cell proliferation during the culture can be appropriately maintained.

The viable cell density can be measured using a commercially available measuring device such as a Cell Viability Analyzer Vi-cell XR manufactured by Beckman Coulter, Inc., and as an example, it can be measured by using Vi-cell XR 2.04 as the Vi-cell software and setting the Min diameter to 6 μm and the Max diameter to 50 μm as parameters at the time of measurement. In addition, the measurement parameters can be set as follows.

Cell brightness: 85%
Cell sharpness: 100
Viable cell spot brightness: 75%
Viable cell spot area: 5%
Minimum circularity: 0
Decluster degree: Medium

In the present invention, it is preferable to perform cell bleeding (also referred to as bleeding) of discharging a culture solution containing cells to an outside of a system and adding a culture medium to maintain the viable cell density substantially constant.

The cell bleeding ratio in the cell bleeding is preferably 0.10 to 0.80 vvd, and more preferably 0.15 to 0.60 vvd.

By setting the cell bleeding ratio to 0.10 vvd or more, the proliferation can be appropriately maintained, and the antibody quality is stably maintained. By setting the cell bleeding ratio to 0.80 vvd or less, the culture medium use amount can be suppressed, and the cost can be reduced.

The bleeding is sufficient to be such that the culture solution containing cells can be extracted from the culture container, and the method thereof is not particularly limited. However, for example, as shown in FIG. 1, a pipe for discharging the culture solution is inserted into the culture solution, and the culture solution can be discharged from the culture container through the pipe.

Preferably, in the bleeding, the culture solution may be bled while an electrostatic capacity of the culture solution is subjected to in-line measurement. The in-line measurement is to measure any measurement value indicating a state of the culture solution while carrying out cell culture. In order to subject the electrostatic capacity to in-line measurement, for example, as shown in FIG. 1, it is sufficient to insert an electrostatic capacity sensor into the culture solution and subject the electrostatic capacity of the culture solution to in-line measurement while carrying out culture.

Preferably, the bleeding may be automatically controlled. The automatic control means that the start or stop of the bleeding is automatically controlled based on any measurement value indicating any state of the culture solution. For example, the start and stop of the bleeding can be automatically controlled based on the measurement value of the electrostatic capacity of the culture solution.

Preferably, the bleeding may be carried out such that a variation of the electrostatic capacity of the culture solution, where the electrostatic capacity is subjected to in-line measurement, is ±20% or less with respect to an average value of an electrostatic capacity during the production period. The variation of the electrostatic capacity of the culture solution, where the electrostatic capacity is subjected to in-line measurement, is more preferably ±15% or less with respect to an average value of an electrostatic capacity during the production period, still more preferably ±12% or less with respect to the average value of the electrostatic capacity during the production period, and particularly preferably ±8% or less with respect to the average value of the electrostatic capacity during the production period. The lower limit of the variation of the electrostatic capacity of the culture solution is not particularly limited, and it is sufficient to be 0% or more; however, a variation of substantially 0.1% or more occurs in consideration of the variation of daily cell proliferation.

One exemplary bleeding can be carried out by a step including:

(i) setting an electrostatic capacity serving as a target;
(ii) setting a weight control value of the culture container;
(iii) measuring the electrostatic capacity of the culture solution at a period of 0.1 seconds or less;
(iv) driving a pump for draining the culture solution at a rate of 0.3 vvd to 3 vvd in a case where the electrostatic capacity of the culture solution exceeds the target (here, vvd means "volume of drained culture solution/volume of culture solution/day");
(v) automatically supplying a culture medium to the culture container while measuring a mass of the culture solution such that a variation of a liquid amount of the culture solution is within 10%; and
(vi) stopping the pump for draining the culture solution in a case where the electrostatic capacity of the culture solution is smaller than the target by 0.01% or more.

FIG. 1 shows one example of a cell culture device that can be used in the cell culture in the present invention. In FIG. 1, a culture container 10 is a container that accommodates a culture solution containing cells. Cells are cultured in the culture solution in the inside of the culture container 10.

Oxygen and air are sent from a sparger air supply pipe 1, and the oxygen and the air are introduced into the culture solution through a sparger 11 having a hole diameter of 20 $\mu$m. The dissolved oxygen concentration inside the culture solution can be adjusted by the sparger 11. The sparger is not particularly limited; however, it is possible to use, for example, a sparger in which an average hole diameter of a gas release unit is 1 $\mu$m or more and 300 $\mu$m or less and which releases a gas containing 30% by volume or more of oxygen.

Air and carbon dioxide are introduced from an air supply pipe 2 to an upper part of the culture solution in the culture container.

The culture medium is supplied to the culture container from a culture medium supply pipe 3. A culture medium supply pump 15 is provided in the culture medium supply pipe 3.

A gas exhaust pipe 4 is a pipe for gas exhaust, and a gas exhaust filter 5 is connected to a terminal of the gas exhaust pipe 4.

A sampling tube 6 is a pipe for collecting (sampling) the culture solution.

A bleeding tube 7 is a pipe for extracting (bleeding) the culture solution.

An electrostatic capacity sensor 8 is mounted to come into contact with the culture solution in the culture container.

A dissolved oxygen sensor 9 is mounted to come into contact with the culture solution in the culture container.

A stirring member having a stirring blade 12 may be provided inside the culture container 10. In a case where the stirring blade 12 is rotated, the culture solution inside the culture container 10 is stirred, and the homogeneity of the culture solution is maintained. In a case where the culture solution is stirred by the stirring blade 12, the bubbles released by the sparger are also stirred. The position of the stirring member having a stirring blade, the size of the stirring blade, and the like are not particularly limited and may be designed depending on the cell kind to be used, the amount of the culture solution, the amount of oxygen to be supplied, or the position, number, size, or the like of the sparger. Further, in order to quickly stir bubbles coming out of the sparger and suppress coalescence of the bubbles, it is preferable to dispose the stirring blade 12 at a position close to the sparger. FIG. 1 shows a state in which foam 19 is present.

A perfusion device 13 is connected to a lower part of the culture container 10. In addition, the culture solution inside the culture container 10 passes through the perfusion device 13 (hollow fiber MF membrane), and the permeated solution containing the cell product is extracted from the culture container 10 by a delivery pump 16. The flow of the permeated solution is indicated by an arrow 14. A pressure gauge 17 and a diaphragm pump 18 may be installed in the perfusion device 13.

In the present invention, a cell suspension extracted from the culture container may be allowed to pass through a separation membrane to separate the cell suspension into a cell-containing liquid and a permeated solution. This operation can be carried out using a perfusion device. In this operation, the cell suspension extracted from the culture container is separated into a cell-containing liquid having a cell density higher than that of the cell suspension and a permeated solution having a cell density lower than that of the cell suspension. The cell density can be measured by a live/dead cell analyzer Vi-CELL XR, manufactured by Beckman Coulter, Inc.

The membrane separation treatment step described above is preferably tangential filtration, more preferably alternating tangential flow (ATF) or tangential flow, and most preferably ATF. Examples of the filter that can perform ATF include SuATF10-S02PES, F2 RF02PES, or the like, manufactured by Repligen Corporation.

As the culture medium that is used for cell culture, a culture medium that is generally used for culturing animal cells can be used. For example, CD OptiCHO (manufactured by Thermo Fisher Scientific, Inc.), Dulbecco's modified Eagle medium (DMEM), Eagle minimum essential medium (MEM), RPMI-1640 medium, RPMI-1641 medium, F-12K medium, Ham's F12 medium, Iscove's modified Dulbecco's medium (IMDM), McCoy's 5A medium, Leibovitz's L-15 medium, and EX-CELL (trade mark) 300 series (JRH Biosciences), CHO-S-SFMII (Invitrogen), CHO-SF (Sigma-Aldrich Co. LLC), CD-CHO (Invitrogen), ISCHO-V (FUJIFILM Irvine Scientific), PF-ACF-CHO (Sigma-Aldrich Co. LLC), and the like can be used. Alternatively, a homemade culture medium may be used.

Serum such as fetal calf serum (FCS) may be added to the culture medium, or serum may not be added thereto. The culture medium may be supplemented with additional components such as an amino acid, salts, a sugar, a vitamin, a hormone, a growth factor, a buffer solution, an antibiotic, a lipid, a trace element, and a hydrolysate of a plant protein. A protein-free culture medium can also be used.

Although the pH of the culture medium varies depending on the cells to be cultured, the culture medium generally has a pH of 6.0 to 8.0, preferably has a pH of 6.4 to 7.6, and more preferably has a pH of 6.7 to 7.4.

The culture temperature is generally 30°C to 40°C, preferably 32°C to 39°C, and more preferably 36°C to 38°C, and the culture temperature may be changed during the culture.

The culture can be carried out in an atmosphere having a $CO_2$ concentration of 0% to 40% by volume, preferably 2 to 25% by volume, and more preferably 3 to 20% by volume.

The amount of the culture solution is preferably 1 L or more, more preferably 50 L or more, still more preferably 100 L or more, and still more preferably 200 L or more.

The culture period is not particularly limited, but it is generally 5 days or more and 300 days or less, preferably 14 days to 200 days or less, more preferably 14 days or more and 100 days or less, and still more preferably 20 days or more and 90 days or less. In the present invention, the culture period is preferably 14 days or more.

In the culture, the culture medium can be replaced, aerated, and stirred as necessary. In a case of carrying out stirring of the culture, the rotation speed of stirring is not particularly limited; however, the stirring power per unit volume is generally 10 to 300 kW/m$^3$, preferably 20 to 200 kW/m$^3$, and more preferably 30 to 100 kW/m$^3$.

The dissolved oxygen concentration in the culture solution can be appropriately set and is not particularly limited; however, it is 20% to 150%, preferably 30% to 120%, and more preferably 50% to 100% in a case where the saturated dissolved oxygen concentration in the liquid at 37°C in air at 1 atm is set to 100%.

The cell culture can be carried out using a cell culture device having the configuration described above in the present specification. The cell culture device may be any one of a fermenter tank type culture device, an air lift type culture device, a culture flask type culture device, a spinner flask type culture device, a microcarrier type culture device, a fluidized bed type culture device, a hollow fiber type culture device, a roller bottle type culture device, a filling tank type culture device, or the like. The culture container is preferably a single-use culture tank from the viewpoint of homogenizing the culture environment or the like. From the viewpoint of homogenization of the culture environment and the like, the cell culture device may be a single-use culture tank.

The viscosity of the culture solution is preferably 1.2 mPa·s or more and less than 15 mPa·s, more preferably 1.4 mPa·s or more and less than 12 mPa·s, and particularly preferably 1.6 mPa·s or more and less than 10 mPa·s.

In the present invention, a pH adjusting agent may be added during the culture. From the viewpoint of the clogging of the filtration filter flow channel, a pH adjusting agent to be added to the culture tank is such that an adding amount thereof per day is preferably 8 mmol/day/L or less and more preferably 7 mmol/day/L or less. Here, it is not necessary to add a pH adjusting agent.

The pH adjusting agent is not particularly limited; however, it is preferably an aqueous $Na_2CO_3$ solution, an aqueous $NaOH$ solution, or an aqueous $NaHCO_3$ solution, and it is more preferably an aqueous $NaHCO_3$ solution. The pH adjusting agent may be added alone or may be added by being mixed with a culture medium or an anti-foaming agent. In order to avoid the local variation of pH in the culture solution due to the addition of the pH adjusting agent, it is preferable that the pH adjusting agent is added by being mixed with a culture medium.

The cell used in the present invention has an isovaleryl-CoA dehydrogenase gene Ivd, but the isovaleryl-CoA dehydrogenase gene Ivd is preferably an endogenous gene. The endogenous gene means a gene that the cell originally has, and is distinguished from a foreign gene introduced from the outside.

Further, according to the present invention, there is provided a production method for a product, including:

a screening step of selecting cells in which a ratio of an expression level of an isovaleryl-CoA dehydrogenase gene Ivd to an expression level of a glycerol-3-phosphate dehydrogenase gene Gpdh is 0.0010 or more and 0.0100 or less; and

a step of culturing the cells selected in the screening step,

in which expression of the isovaleryl-CoA dehydrogenase gene Ivd of the cells is endogenous gene expression.

The kind of the cell in the present invention is not particularly limited; however, examples thereof include eukaryotic cells such as an animal cell, a plant cell, and yeast, prokaryotic cells such as Bacillus subtilis, and Escherichia coli. The cell is preferably an animal cell (more preferably a mammalian cell) or an insect cell, and it is most preferably a mammalian cell. The cell may be a primary cell or a cell established as a cell line.

Examples of the cell include a Chinese hamster ovary (CHO) cell, a HEK cell (a cell derived from the human embryonic kidney), a BHK cell, a 293 cell, a C127 cell, a myeloma cell (such as an NS0 cell), a PerC6 cell, an SP2/0 cell, a hybridoma cell, a COS cell (a cell derived from the kidney of the African green monkey), a 3T3 cell, a HeLa cell, a Vero cell (a renal epithelial cell of the African green monkey), a MDCK cell (a cell derived from a canine kidney renal tubular epithelial cell), a PC12 cell, and a WI38 cell. The cell may be a stem cell such as an embryonic stem cell (ES cell) or an induced pluripotent stem cell (iPS cell). Among these, a CHO cell, a HEK cell, a BHK cell, or a hybridoma is preferable, where a CHO cell or a HEK cell is more preferable, and a CHO cell is most preferable. The CHO cell is widely used for the production of recombinant proteins such as a cytokine, a coagulation factor, and an antibody. It is preferable to use a CHO cell deficient in dihydrofolate reductase (DHFR), and as a DHFR-deficient CHO cell, it is possible to use, for example, CHO-DG44.

It is preferable that the cell viability is high; however, it is preferably 80% or more, more preferably 85% or more, particularly preferably 90% or more, and most preferably 95% or more.

These cells may be cells into which a foreign gene encoding a protein desired to be expressed (for example, an antibody) has been introduced. The cell is preferably a cell that produces an antibody. An expression vector can be used for introducing a foreign gene encoding a protein desired to be expressed, into a cell. An expression vector containing a DNA encoding a protein desired to be expressed, an expression control sequence (for example, an enhancer, a promoter, a terminator, or the like), and a selection marker gene as desired is introduced into a cell, whereby it is possible to prepare a cell into which a foreign gene encoding a protein desired to be expressed is introduced. The expression vector is not particularly limited and can be appropriately selected and used depending on the kind, use application, and the like of the cell.

As the promoter, it is possible to use any promoter of which the function can be exhibited in mammalian cells. Examples thereof include a promoter of the immediate early (IE) gene of the cytomegalovirus (CMV), an early promoter of SV40, a retrovirus promoter, a metallothionein promoter, a heat shock promoter, an SRα promoter, and a promoter and enhancer of the Moloney murine leukemia virus. In addition, an enhancer of the IE gene of human CMV may be used together with the promoter.

As the selection marker gene, it is possible to use, for example, a drug resistance gene (a neomycin resistance gene, a DHFR gene, a puromycin resistance gene, a blasticidin resistance gene, a hygromycin resistance gene, a cycloheximide resistance gene), or a fluorescence gene (a gene encoding a green fluorescent protein GFP or the like).

The method of introducing an expression vector into a cell is not particularly limited, and it is possible to use, for example, a calcium phosphate method, an electroporation method, a liposome method, a gene gun method, or a lipofection method.

The production method for a product according to the embodiment of the present invention includes culturing a cell using a cell culture device to produce a product from the cell.

According to the present invention, there is provided a product that is produced by the production method for a product according to the embodiment of the present invention.

In the present invention, the kind of product is not particularly limited; however, the product is preferably a protein. Examples of the product include a recombinant polypeptide chain, a recombinant secreted polypeptide chain, an antigen-binding protein, an antibody (for example, a human antibody, a humanized antibody, a chimeric antibody, a mouse antibody, or a bispecific antibody), an Fc fusion protein, a fragmented immunoglobulin, and a single-chain antibody (scFv).

In addition, it may be, in addition to the above, an adenovirus, an adeno-associated virus, a lentivirus, or the like.

The product is preferably an antibody, and more preferably a human antibody, a humanized antibody, a chimeric antibody, or a mouse antibody. Examples of the fragmented immunoglobulin include Fab, F(ab')2, and Fv. The class of the antibody is also not particularly limited, and it may be any class of IgG such as IgG1, IgG2, IgG3, or IgG4, IgA, IgD, IgE, or IgM. However, IgG or IgM is preferable in a case of being used as a medicine.

The human antibody includes all antibodies having one or a plurality of variable and constant regions induced from human immunoglobulin sequences. In one embodiment, all variable and constant domains are induced from human immunoglobulin sequences (complete human antibodies).

In a case of being administered to a human subject, the humanized antibody has a sequence different from a sequence of an antibody induced from a non-human species by substitution, deletion, and/or addition of one or a plurality of amino acids so that there is a low possibility for the humanized antibody to induce an immune response and/or so that induction of a severe immune response is reduced as compared with the antibody of non-human species. In one example, specific amino acids in a framework and constant domains of heavy chains and/or light chains of an antibody of non-human species are mutated to produce a humanized antibody. In another example, a constant domain from a human antibody is fused to a variable domain of an antibody of a non-human species.

The chimeric antibody is an antibody in which variable regions and constant regions having origins different from each other are linked. For example, an antibody consisting of variable regions of heavy chains and light chains of a mouse antibody and consisting of constant regions of heavy chains and light chains of a human antibody is a mouse/human heterologous chimeric antibody. It is possible to prepare a recombinant vector expressing a chimeric antibody by linking a DNA encoding variable regions of a mouse antibody and a DNA encoding constant regions of a human antibody and then incorporating the linked DNA into an expression vector. It is possible to acquire a chimeric antibody produced during the culturing by culturing a recombinant cell transformed with the above vector and expressing the incorporated DNA.

The bispecific antibody is an antibody that recognizes two kinds of antigenic specificity, which are different from each other. Various forms of bispecific antibodies are present. As a method of preparing a bispecific antibody, it has been reported a method of preparing a bispecific antibody by binding two immunoglobulin molecules by using a crosslinking agent such as N-succinimidyl 3-(2-pyridyldithiol)propionate or S-acetylmercaptosuccinic acid anhydride, a method of preparing a bispecific antibody by binding Fab fragments of immunoglobulin molecules to each other, and the like. In addition, a bispecific antibody can be expressed by introducing a gene encoding the bispecific antibody into a cell.

The Fc fusion protein indicates a protein having an Fc region and includes an antibody. The Fab is a monovalent fragment having VL, VH, CL, and CH1 domains.

The F(ab')2 is a divalent fragment having two Fab fragments bound by a disulfide crosslinking at a hinge region.

The Fv fragment has VL and VH domains of a single arm of an antibody.

The single-chain antibody (scFv) is an antibody in which VL and VH regions are joined through a linker (for example, a synthetic sequence of amino acid residues) to form a continuous protein chain, where the linker is long enough to allow the protein chain to fold for itself and form a monovalent antigen binding site.

The antibody is not particularly limited; however, examples thereof include an anti-IL-6 receptor antibody, an anti-IL-6 antibody, an anti-glypican-3 antibody, an anti-CD3 antibody, an anti-CD20 antibody, an anti-GPIIb/IIIa antibody, an anti-TNF antibody, an anti-CD25 antibody, an anti-EGFR antibody, an anti-Her2/neu antibody, an anti-RSV antibody, an anti-CD33 antibody, an anti-CD52 antibody, an anti-IgE antibody, an anti-CD11a antibody, an anti-VEGF antibody, and an anti-VLA4 antibody.

Regarding the product recovery, the culture solution may be simply recovered. For example, a liquid obtained by removing at least a part of the cells from the culture solution using a filter or a centrifuge may be recovered, and a known method is used without particular limitation. In a case of desiring to improve the purity of the product, change the solvent of the product, or change the form of the product to, for example, a powder form, the culture solution or the liquid can be subjected to further treatment.

In addition, a part of the culture solution can be recovered while being perfused, or a part of the culture solution can be recovered as a liquid which is obtained by removing at least a part of cells from the part of the culture solution, which is recovered by using a filter or a centrifuge while being perfused.

The product can be purified by a purification treatment. The obtained product can be purified to high purity. For the separation and purification of the product, the separation and purification methods that are used for general proteins may be used. For example, it is possible to carry out separation and purification of a product by appropriately selecting and combining means such as a chromatography such as affinity chromatography, a filter, ultrafiltration, salting out, dialysis, sodium dodecyl sulfate (SDS) polyacrylamide gel electrophoresis, isoelectric focusing electrophoresis, and the like, which are not limited thereto. The concentration of the product obtained as above can be measured according to absorbance measurement, enzyme-linked immunosorbent assay (ELISA), or the like. In addition, in a case where the product is an antibody, the titer of the antibody can also be measured with a commercially available analytical instrument such as Cedex Bio manufactured by F. Hoffmann-La Roche, Ltd.

Examples of the column that is used for affinity chromatography include a protein A column and a protein G column. Examples of the chromatography other than affinity chromatography include ion exchange chromatography, hydrophobic chromatography, gel filtration, reverse phase chromatography, and adsorption chromatography. The chromatography can be carried out using liquid phase chromatography such as high performance liquid chromatography (HPLC) or fast protein liquid chromatography (FPLC).

It is noted that it is also possible to modify the product or partially remove a peptide thereof by allowing an appropriate polypeptide modifying enzyme to act on the useful substance before or after purification. As the polypeptide modifying enzyme, for example, trypsin, chymotrypsin, lysyl endopeptidase, protein kinase, or glucosidase is used.

The product that is produced according to the present invention can be used, for example, for a biopharmaceutical product, or regenerative medicine.

The present invention will be more specifically described using the following examples; however, it is not limited by the examples.

Examples

<Establishment of antibody-producing cell>

A vector containing a nucleic acid sequence encoding IgG1 was constructed, and the constructed vector was introduced into CHO-DG44 cells, whereby CHO-DG44 cells expressing IgG1 (IgG1 cells) were prepared. The prepared cells were screened, and three types of cells of CHO cell A, CHO cell B, and CHO cell C were used for the experiment. The construction of the CHO vector and the introduction thereof into the cell were carried out according to Example 2 of JP2016-517691A. As described above, CHO cells producing monoclonal antibodies were prepared and used in the following experiment.

[Perfusion culture experiment]

In Examples and Comparative Examples, experiments were carried out using the cell culture device shown in FIG. 1. CHO cells that produce antibodies in perfusion culture were cultured to a target cell density, and the culture was continued while maintaining the cell density. The culture was continued for a total of 34 days as long as the cell proliferation capacity did not decrease and the target cell density could be maintained. In a case where the cell proliferation capacity decreased and the target cell density was not reached, or in a case where after the target cell density was reached, a period during which the cell bleeding was not performed continued for 1 day and the cell density could not be maintained, the culture was stopped.

Specifically, the following experiments were performed.

1.3 L of a culture medium is charged into a culture tank having a total capacity of 3 L (Biostat (registered trademark)) manufactured by Sartorius AG. The culture medium is a culture medium adjusted with reference to the exchange culture medium in Table 2 of JP2000-517188A. CHO cells were seeded in the above culture medium at $0.5 \times 10^6$ cells/ml. At a stirring rotation speed of 160 rpm, 38 ml/min of air and 2 ml/min of $CO_2$ from the upper surface, and $O_2$ from the bottom surface were automatically controlled and supplied from a sparger installed in the culture tank so that the oxygen concentration in the culture solution was 80%.

After culturing for 2 days after seeding, the cell culture solution was continuously filtered with ATF2 manufactured by Repligen Corporation, and the recovery liquid was recovered while continuously supplying a culture medium at a perfusion ratio of 0.6 vvd.

Further, on the 5th day, the perfusion ratio was changed to the predetermined perfusion ratio shown in Table 2, and the perfusion ratio was maintained thereafter.

Thereafter, in a case where the cell density reached the target cell density shown in Table 2, the cell bleeding was carried out while a part of the culture solution was drained so that the cell density was maintained. The cell bleeding was continuously carried out automatically such that the cell density was constant using a FUTURA sensor manufactured by ABER Instruments Ltd. while monitoring the electrostatic capacity of the culture solution.

In this case, a plurality of levels were performed by changing the cells used, the culture medium, the culture conditions, and the like as shown in Examples and Comparative Examples of Table 2.

Thereafter, the culture was continued, and the evaluation described below was carried out.

In a case of the 50 L culture, the culture was carried out under the same conditions as those in the 1.3 L culture, except that 50 L of a culture medium was charged into a culture tank of Hyperforma 50 manufactured by Thermo Fisher Scientific Inc., the stirring rotation speed was set to 250 rpm, the setting was such that an upper surface air of 0.475 L/min and $CO_2$ of 0.025 L/min, and ATF4 manufactured by Repligen Corporation was used.

<Automatic control method for cell bleeding>

In a case where the cell bleeding was automatically controlled, the cell bleeding was continuously carried out automatically using a FUTURA sensor manufactured by ABER Instruments Ltd. while monitoring the electrostatic capacity of the culture solution. Specifically, the measurement was carried out by the following method.

(1) An electrostatic capacity serving as a target is set.
(2) A weight control value of the culture tank is set.
(3) The electrostatic capacity of the culture solution is measured at a period of 0.1 s or less.
(4) A pump for draining the culture solution is driven at a rate of 0.3 vvd to 3 vvd in a case where the electrostatic capacity of the culture solution exceeds the target.
(5) A culture medium is automatically supplied into the culture tank while the weight of the culture tank is measured so that the liquid amount of the culture solution is roughly kept constant.
(6) The pump for draining the culture solution is stopped in a case where the electrostatic capacity of the culture solution is smaller than the target by 0.01% or more.

<Measurement and evaluation method>

(1) Measurement of cell density

The culture solution in the culture tank was extracted and subjected to measurement using a Cell Viability Analyzer Vi-cell XR manufactured by Beckman Coulter, Inc. It is noted that for the Vi-cell software, Vi-cell XR2.04 was used, and the parameters at the time of measurement were set as follows.

Min diameter: 6 $\mu$m
Max diameter: 50 $\mu$m
Dilution: In a case where the cell density was $100 \times 10^5$ cells/mL or less, the sample was not diluted, and the measurement was carried out at a Dilution of 1. In a case where the cell density was $100 \times 10^5$ cells/mL or more, the sample was diluted 10 times, and the measurement was carried out at a Dilution of 10.
Cell brightness: 85%
Cell sharpness: 100
Viable cell spot brightness: 75%
Viable cell spot area: 5%
Minimum circularity: 0
Decluster degree: Medium

The measurement was carried out once a day by the above method.

(2) Measurement of antibody concentration in culture solution and permeated solution

A sample of the culture solution was extracted from the culture tank, and the cells were filtered with Whatman (pore size: 0.2 $\mu$m, diameter: 25 mm) manufactured by Cytiva. Then, the filtrate was measured with Cedex Bio manufactured by F. Hoffmann-La Roche, Ltd.
The permeated solution was measured with Cedex Bio manufactured by F. Hoffmann-La Roche, Ltd. after sampling.
The measurement was carried out once a day by the above method.

(3) Measurement of mannose 5 (M5) as quality of antibody

The profile of the N-type sugar chain including M5 was measured by the following method.
The antibody in the antibody recovered liquid obtained was subjected to a digestion treatment with peptide-N-glycosidase F to cut out the N-type sugar chain. Thereafter, the cut sugar chain was subjected to fluorescence labeling with 2-aminopyridine. Then, the mannose 5 was subjected to quantitative analysis by reverse phase high performance liquid chromatography (HPLC) to calculate the peak of mannose 5 with respect to the total peak area.

(4) Measurement of isovaleric acid concentration in culture solution

A sample of the culture solution was extracted from the culture tank, the cells were filtered with Whatman (pore size: 0.2 $\mu$m, diameter: 25 mm) manufactured by Cytiva, and the content of isovaleric acid was measured in the filtrate using chromatographic separation using ICS-2100 and IonPac AS11-HC column which are manufactured by Dionex.

(5) Measurement of ratio X of expression level of isovaleryl-CoA dehydrogenase gene Ivd to expression level of glycerol-3-phosphate dehydrogenase gene Gpdh in culture solution

Cells in the culture solution were treated with RNeasy plus mini kit (Qiagen) to harvest Total RNA. Reverse transcription was performed on the obtained Total RNA using PrimeScriptTM RT Master Mix (Perfect Real Time, Takara). Primers homologous to the endogenous IVd (SEQ ID NOS: 1 and 2) and primers to the glycerol-3-phosphate dehydrogenase Gpdh gene (SEQ ID NOS: 3 and 4) as an internal standard were designed and real-time PCR (polymerase chain reaction) using SYBR (registered trademark) Premix Ex TaqTM (Tli RNaseH Plus, Takara) was performed. SEQ ID NOS 1 to 4 are shown in Table 1.

The Gpdh gene was used for standardization, and the expression level of the Ivd gene was confirmed.

[Table 1]

| SEQ ID | Primer | Base sequence |
| --- | --- | --- |
| 1 | Ivd-Forward | AGGTACCCAACGCCTTTTCT |
| 2 | Ivd-Reverse | GAGGCCAGCAAGAGACAATC |
| 3 | Gapdh-Forward | TGGTGAAGCAGGCATCTGAG |
| 4 | Gapdh-Reverse | GTCCAGGGTGGGCTTCTTAC |

<Evaluation determination>

On the last culture day, the proliferation capacity and the mannose 5 (M5) as the antibody quality were evaluated and determined, and the overall evaluation was performed. In a case where the cell proliferation capacity decreased and the target cell density was not reached, or in a case where after the target cell density was reached, a period during which the cell bleeding was not performed continued for 1 day and the target cell density could not be maintained, the culture was stopped. Table 2 shows the measurement results on the last culture day.

(1) Proliferation capacity

D: The target cell density was not reached, or the target cell density was reached but the period during which cell bleeding was not performed continued for 1 day within 34 days after the start of culture and 120M could not be maintained, and the culture was stopped
B: The culture is continued until the 34th day after the start of culture, and the cell bleeding rate on the 34th day is less than 0.10 vvd
A: In a case where the culture is continued until the 34th day after the start of culture, and the cell bleeding rate on the 34th day is 0.10 vvd or more

(2) Mannose 5 (M5) as antibody quality

C: In a case where mannose 5 is 7% or more
B: In a case where mannose 5 is 4% or more and less than 7%
A: In a case where mannose 5 is less than 4%

(3) Overall evaluation

D: In a case where there is a D determination
C: In a case where there are no D determination and one or more C determinations
B: In a case where all are B determination or more and there is one or less A determination
A: In a case where all are B determination or more and there are two A determinations

Table 2 shows measurement results of each of Examples and each of Comparative Examples.

[Flask batch culture experiment]

In order to confirm that cells having a large Ivd gene expression level can be screened in advance by flask culture, batch culture in a flask was also performed in parallel with the above-described perfusion culture.

Specifically, the following experiments were performed.

The same cells as the cells used in the perfusion culture were seeded in a flask having a capacity of 125 mL at a seeding density of $0.5 \times 10^6$ cells/ml such that the liquid amount was 30 ml, and shaking cultured at 37°C and at a rate of 140 rpm in a 5% $CO_2$ atmosphere. The culture solution on the 4th day after seeding was sampled, and the ratio X of expression level of isovaleryl-CoA dehydrogenase gene Ivd to expression level of glycerol-3-phosphate dehydrogenase gene Gpdh in culture solution was measured according to the following procedure.

Cells in the culture solution were treated with RNeasy plus mini kit (Qiagen) to harvest Total RNA. Reverse transcription was performed on the obtained Total RNA using PrimeScriptTM RT Master Mix (Perfect Real Time, Takara). Primers homologous to the endogenous IVd (SEQ ID NOS: 1 and 2) and primers to the glycerol-3-phosphate dehydrogenase Gpdh gene (SEQ ID NOS: 3 and 4) as an internal standard were designed and real-time PCR (polymerase chain reaction) using SYBR (registered trademark) Premix Ex TaqTM (Tli RNaseH Plus, Takara) was performed. The Gpdh gene was used for standardization, and the expression level of the Ivd gene was confirmed. Table 2 shows measurement results of each of Examples and each of Comparative Examples.

[Table 2]

| | Culture conditions | | | | Results of flask culture | Results of Perfusion culture | | | | | | | | | Evaluations | | |
|---|---|---|---|---|---|---|---|---|---|---|---|---|---|---|---|---|---|
| | Amount of culture solution [L] | Type of CHO cell | Perfusion rate P after 5th day [vvd] | Target viable cell density [M cells/ml] | X | Whether target viable cell density is reached | Last culture day | Viable cell density V [M cells/ml] | X | X · PV | Isovaleric acid concentration [µM] | Cell bleeding rate [vvd] | MS [%] | Titer [g/L day] | Proliferation | MS | Overall evaluation |
| Example 1 | 1.3 | A | 1.2 | 120 | 0.0022 | Reached | 34 | 135 | 0.0034 | 3.3E-05 | 196 | 0.43 | 3 | 1.7 | A | A | A |
| Example 2 | 1.3 | B | 1.2 | 120 | 0.0014 | Reached | 34 | 122 | 0.0025 | 2.5E-05 | 514 | 0.30 | 4 | 1.7 | A | B | B |
| Example 3 | 1.3 | B | 0.8 | 100 | 0.0014 | Reached | 34 | 101 | 0.0019 | 1.5E-05 | 840 | 0.09 | 7 | 1.7 | B | C | C |
| Example 4 | 1.3 | B | 1.8 | 120 | 0.0014 | Reached | 34 | 135 | 0.0037 | 3.9E-05 | 311 | 0.28 | 3 | 1.8 | A | A | A |
| Example 5 | 1.3 | B | 2 | 180 | 0.0014 | Reached | 34 | 176 | 0.0037 | 3.1E-05 | 611 | 0.18 | 5 | 2.7 | A | B | B |
| Example 6 | 1.3 | C | 2 | 120 | 0.0008 | Reached | 34 | 121 | 0.0011 | 1.8E-05 | 787 | 0.11 | 6 | 1.7 | B | B | B |
| Example 7 | 50 | B | 1.2 | 120 | 0.0014 | Reached | 34 | 130 | 0.0028 | 2.8E-05 | 498 | 0.34 | 4 | 1.7 | A | B | B |
| Comparative Example 1 | 1.3 | C | 1.2 | 120 | 0.0008 | Reached | 18 | 110 | 0.0009 | 9.8E-06 | 1060 | 0.00 | 7 | 1.8 | D | C | D |
| Comparative Example 2 | 1.3 | C | 0.8 | 100 | 0.0008 | Reached | 17 | 98 | 0.0009 | 7.3E-06 | 1240 | 0.00 | 8 | 1.4 | D | C | D |

Explanation of References

1: sparger air supply pipe
2: air supply pipe
3: culture medium supply pipe
4: gas exhaust pipe
5: gas exhaust filter
6: sampling tube
7: bleeding tube
8: electrostatic capacity sensor
9: dissolved oxygen sensor
10: culture container
11: sparger
12: stirring blade
13: perfusion device
14: flow of permeated solution
15: culture medium supply pump
16: delivery pump
17: pressure gauge
18: diaphragm pump
19: foam

[Sequence list]

International application 23F00559W1JP24019990_2.xml based on International Patent Cooperation Treaty

**Claims**

**1.** A production method for a product, comprising:

performing perfusion culture of cells in a culture solution,
wherein a perfusion ratio P (vvd) in the perfusion culture,
a cell density V ($\times 10^6$ cells/ml) in the culture solution, and
X, which is a ratio of an expression level of an isovaleryl-CoA dehydrogenase gene Ivd to an expression level of a glycerol-3-phosphate dehydrogenase gene Gpdh in the culture solution, satisfy

$$1.0 \times 10^{-5} \leq X \times (P/V) \leq 1.0 \times 10^{-4},$$

**2.** The production method for a product according to claim 1,
wherein an isovaleric acid concentration in the culture solution is 10 $\mu$mol/L or more and 1,000 $\mu$mol/L or less.

**3.** The production method for a product according to claim 1,
wherein X is 0.0010 or more and 0.0100 or less.

**4.** The production method for a product according to any one of claims 1 to 3,
wherein the perfusion culture includes producing the product in a certain period while maintaining a target viable cell density substantially constant after reaching the viable cell density in the perfusion culture.

**5.** The production method for a product according to claim 4,
wherein the viable cell density maintained substantially constant is 30 $\times 10^6$ cells/ml or more and 400 $\times 10^6$ cells/ml or less.

**6.** The production method for a product according to claim 4,
wherein the perfusion ratio is 0.3 or more and 5.0 or less in a period in which the viable cell density is maintained substantially constant.

7. The production method for a product according to claim 4,
wherein cell bleeding of discharging a culture solution containing cells to an outside of a system and adding a culture medium is performed to maintain the viable cell density substantially constant.

8. The production method for a product according to claim 7,
wherein a cell bleeding ratio in the cell bleeding is 0.10 to 0.80 vvd.

9. The production method for a product according to any one of claims 1 to 3,
wherein a period of the perfusion culture is 14 days or more and 200 days or less.

10. The production method for a product according to any one of claims 1 to 3,
wherein the isovaleryl-CoA dehydrogenase gene Ivd is an endogenous gene.

11. The production method for a product according to any one of claims 1 to 3,
wherein the cell is a CHO cell.

12. The production method for a product according to any one of claims 1 to 3,
wherein the product is a protein.

13. The production method for a product according to claim 12,
wherein the protein is an antibody.

14. A production method for a product, comprising:

a screening step of selecting cells in which a ratio of an expression level of an isovaleryl-CoA dehydrogenase gene Ivd to an expression level of a glycerol-3-phosphate dehydrogenase gene Gpdh is 0.0010 or more and 0.0100 or less; and
a step of culturing the cells selected in the screening step,
wherein expression of the isovaleryl-CoA dehydrogenase gene Ivd of the cells is endogenous gene expression.

## FIG. 1

## INTERNATIONAL SEARCH REPORT

| | International application No. |
|---|---|
| | **PCT/JP2024/019990** |

**A.    CLASSIFICATION OF SUBJECT MATTER**

*C12P 21/02*(2006.01)i; *C12N 15/53*(2006.01)i; *C12P 21/08*(2006.01)i
FI:    C12P21/02 C; C12P21/08 ZNA; C12N15/53

According to International Patent Classification (IPC) or to both national classification and IPC

**B.    FIELDS SEARCHED**

Minimum documentation searched (classification system followed by classification symbols)

C12P21/02; C12N15/53; C12P21/08

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C.    DOCUMENTS CONSIDERED TO BE RELEVANT**

| Category* | Citation of document, with indication, where appropriate, of the relevant passages | Relevant to claim No. |
|---|---|---|
| A | WO 2019/117136 A1 (FUJIFILM CORPORATION) 20 June 2019 (2019-06-20) | 1-14 |
| A | WO 2006/001473 A1 (JAPAN SCIENCE & TECHNOLOGY AGENCY) 05 January 2006 (2006-01-05) | 1-14 |
| A | WO 2004/029248 A1 (SUMITOMO PHARMACEUTICALS CO., LTD.) 08 April 2004 (2004-04-08) | 1-14 |
| A | ANTONOVA, Olga et al. Expression profiling of muscle invasive and non-invasive bladder tumors for biomakers identification related to drug resistance, sensitivity and tumor progression. Biotechnology & Biotechnological Equipment. 2020, vol. 34, pp. 506-514 | 1-14 |
| A | WO 2019/181234 A1 (FUJIFILM CORPORATION) 26 September 2019 (2019-09-26) | 1-14 |
| A | JP 2021-505182 A (AMGEN INC.) 18 February 2021 (2021-02-18) | 1-14 |

☐ Further documents are listed in the continuation of Box C.    ☑ See patent family annex.

| | |
|---|---|
| *    Special categories of cited documents:<br>"A"    document defining the general state of the art which is not considered to be of particular relevance<br>"D"    document cited by the applicant in the international application<br>"E"    earlier application or patent but published on or after the international filing date<br>"L"    document which may throw doubts on priority claim(s) or which is cited to establish the publication date of another citation or other special reason (as specified)<br>"O"    document referring to an oral disclosure, use, exhibition or other means<br>"P"    document published prior to the international filing date but later than the priority date claimed | "T"    later document published after the international filing date or priority date and not in conflict with the application but cited to understand the principle or theory underlying the invention<br>"X"    document of particular relevance; the claimed invention cannot be considered novel or cannot be considered to involve an inventive step when the document is taken alone<br>"Y"    document of particular relevance; the claimed invention cannot be considered to involve an inventive step when the document is combined with one or more other such documents, such combination being obvious to a person skilled in the art<br>"&"    document member of the same patent family |

| Date of the actual completion of the international search | Date of mailing of the international search report |
|---|---|
| **02 August 2024** | **13 August 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**

| International application No. |
|---|
| **PCT/JP2024/019990** |

---

**Box No. I     Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

        ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

---

Form PCT/ISA/210 (continuation of first sheet) (July 2022)

## INTERNATIONAL SEARCH REPORT
### Information on patent family members

International application No.

**PCT/JP2024/019990**

| Patent document cited in search report | | | Publication date (day/month/year) | Patent family member(s) | | | Publication date (day/month/year) |
|---|---|---|---|---|---|---|---|
| WO | 2019/117136 | A1 | 20 June 2019 | US | 2020/0299723 | A1 | |
| | | | | EP | 3725877 | A1 | |
| | | | | CN | 111465686 | A | |
| WO | 2006/001473 | A1 | 05 January 2006 | US | 2007/0128720 | A1 | |
| | | | | EP | 1783209 | A1 | |
| WO | 2004/029248 | A1 | 08 April 2004 | US | 2008/0014636 | A1 | |
| | | | | EP | 1557466 | A1 | |
| WO | 2019/181234 | A1 | 26 September 2019 | US | 2020/0399585 | A1 | |
| | | | | EP | 3770266 | A1 | |
| | | | | CN | 111868249 | A | |
| JP | 2021-505182 | A | 18 February 2021 | US | 2021/0163592 | A1 | |
| | | | | WO | 2019/118426 | A1 | |
| | | | | EP | 3724229 | A1 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021505182 A
- WO 2019117136 A
- JP 2016517691 A
- JP 2000517188 A